(19) Europäisches Patentamt
European Patent Office
Office européen des brevets

(11) Publication number: 0 213 701 A1

# (12) EUROPEAN PATENT APPLICATION

(21) Application number: 86305240.3

(22) Date of filing: 07.07.86

(51) Int. Cl.⁴: C07C 149/20 , C07D 251/34 , G03F 7/10

(30) Priority: 16.07.85 US 755511

(43) Date of publication of application: 11.03.87 Bulletin 87/11

(84) Designated Contracting States: AT BE CH DE FR GB IT LI LU NL SE

(71) Applicant: W.R. GRACE & CO.
Grace Plaza 1114 Avenue of the Americas
New York New York 10036(US)

(72) Inventor: Morgan, Charles Robert
19108, Holberton Lane
Brookeville Maryland 20729(US)
Inventor: Kyle, David Ray
5295 Ribendell, Apt. 3
Columbia Maryland 21044(US)

(74) Representative: Collier, Jeremy Austin Grey et al
J.A.Kemp & Co. 14, South Square Gray's Inn
London WC1R 5EU(GB)

## (54) Thioether containing mercaptocarboxylic acids and process therefor.

(57) Novel thioether containing mercaptocarboxylic acids are prepared by the free radical addition of a polythiol, $(HS)_xR$, wherein R is an organic moiety with the valence of x and x is two or more, across the double bond of an unsaturated carboxylic acid selected from the group consisting of acrylic, methacrylic, itaconic and maleic at a temperature in the range of about 40 to 200°C. These thioether compounds improve the water developability and adhesiveness in photocurable compositions.

EP 0 213 701 A1

# THIOETHER CONTAINING MERCAPTOCARBOXYLIC ACIDS AND PROCESS THEREFOR

This invention relates to novel, thioether containing, mercaptocarboxylic acids employable in thermal or radiation curable compositions to improve water developability and adhesiveness. The invention also relates to a process for forming the thioether containing mercaptocarboxylic acids.

It is known to form thioether containing acids. See U.S Patent No. 4,135,047. However, these materials do not have thiol groups present for subsequent reactions in photochemical systems.

The present invention provides thioether containing mercaptocarboxylic acids, which on addition to a photocurable composition improve its water developability and adhesiveness to substrates and a process for their production.

The invention relates to thioether containing mercaptocarboxylic acids prepared by the free radical addition of polythiol across the double bond of an unsaturated carboxylic acid selected from the group consisting of acrylic, methacrylic, itaconic and maleic.

The polythiols employed herein are of the formula of $(HS)_xR$ wherein R is an organic moiety with the valence of x and x is 2 or more.

Polythiols used herein include simple or complex organic compounds having a multiplicity of pendant or terminally positioned -SH functional groups per average molecule.

The polythiol must contain two or more -SH groups/molecule and have a viscosity range of essentially 0 to 20 million centipoises (cps) at 70°C as measured by a Brookfield Viscometer either alone or when in the presence of an inert solvent, aqueous dispersion or plasticizer. Operable polythiols in the instant invention usually have molecular weights in the range from about 94 to about 20,000, and preferably from about 94 to about 5,000.

The polythiols operable in the instant invention may be exemplified by the general formula $R(SH)_x$ where x is at least 2 and R is a polyvalent organic moiety free from reactive carbon-to-carbon unsaturation. Thus, R may contain cyclic groupings and hetero atomns such as N, P or O and primarily contain carbon-to-carbon unsaturation.

One class of polythiols operable herein are esters of thiol containing acids of the formula HS-R-COOH where R is an organic moiety containing no reactive carbon-to-carbon unsaturation, with polyhydroxy compounds of structure $R_2OH_n$ where $R_2$ is an organic moiety containing no reactive carbon-to-carbon unsaturation and n is 2 or greater. These components will react under suitable conditions to give a polythiol having the general structure:

$$R_2-(O\overset{\overset{O}{\|}}{C}-R-SH)_n$$

where $R_1$ and $R_2$ are organic moieties containing no reactive carbon-to-carbon unsaturation and n is 2 or greater.

Polythiols such as the aliphatic monomeric polythiols (ethane dithiol, hexamethylene dithiol, decamethylene dithiol, dipentaerythritol hexakis-(beta-mercaptopropionate) and aromatic monomeric polythiols such as tolylene-2,4-dithiol, and the like) and some polymeric polythiols such as a thiol-terminated ethylcyclohexyl dimercaptan polymer and the like, and similar polythiols, which are conveniently and ordinarily synthesized on a commercial basis, are operable as a reactant herein. Examples of the polythiol compounds preferred are esters of thioglycolic acid ($HS-CH_2COOH$), alpha-mercaptopropionic acid ($HS-CH(CH_3)-COOH$) and beta-mercaptopropionic acid ($HS-CH_2CH_2COOH$) with polyhydroxy compounds such as glycols, triols, tetraols, pentaols, hexaols and the like. Specific examples of the preferred polythiols include, but are not limited to, ethylene glycol bis (thioglycolate), ethylene glycol bis (beta-mercaptopropionate), trimethylolpropane tris - (thioglycolate), trimethylol-propane tris (beta-mercaptopropionate), pentaerythritol tetrakis - (thioglycolate) and pentaertythritol tetrakis (beta-mercaptopropionate), all of which are commercially available. A specific example of a preferred polymeric polythiol is polypropylene ether glycol bis - (beta-mercaptopropionate) which is prepared from polypropylene-ether glycol (e.g., Pluracol P201, Wyandotte Chgemical Corp.) and beta-mercaptopropionic acid by esterification.

Additionally, non-ester containing polythiols in general and particularly tris-mercaptoethyl isocyanurate described in U. S. 3,676,440 and a mercaptan-terminated polyoxyalkyline thiol commercially available as Capcure 3-800 are also operable reactants.

The unsaturated carboxylic acids used herein are selected from the group consisting of acrylic, methacrylic, itaconic and maleic acid.

The polythiol is reacted with the unsaturated carboxylic acid in the presence of a free radical initiator at temperatures in the range 40 -200°C, preferably 80 -150°C, to form the corresponding thioether containing mercaptocarboxylic acid. The resultant product, if necessary, is purified by distillation or crystallization which methods are well known to one skilled in the art.

Examples of free radical initiators operable herein include azo compounds, organic peroxides, pinacols and the like. The free radical initiator is added to the composition in amounts ranging from 0.01 -10%, preferably 0.1 - 2%, by weight based on the combined weights of the polythiol and the unsaturated carboxylic acid.

The unsaturated carboxylic acid is added to the reaction in an amount ranging from that sufficient to react with one up to all but one of the thiol groups present in the polythiol. It is necessary that the resulatant thioether contain at least one remaining SH group in order for it to take part in a subsequent photocuring reaction.

If desired, the reaction can be carried out in a solvent. Operable solvent should be free of reactive, ethylenic unsaturation and have a boiling point higher than the reaction temperature. Such solvents include, but are not limited to, aromatic hydrocarbons, e. g., benzene, toluene, xylene and aliphatic hydrocarbons such as hexane, cyclohexane, heptane and the like.

In carrying out the instant invention, the preferred method of operation is to add the free radical initiator to the polythiol compound which is then added to the unsaturated carboxylic acid which may or may not be preheated to the desired reaction temperature, either alone or admixed with a solvent. Heating is continued until the corresponding thioether is formed.

The following examples will aid in explaining, but specifically not limit, the instant invention. Unless otherwise noted, all parts and percentages are by weight.

Example 1

488 g of pentaerythritol tetrakis(beta-mercaptopropionate), 130 g of itaconic acid and 6.1 g of benzopinacol were charged to a 2,000 ml, 3-neck, round bottom flask containing 700 ml benzene and equipped with condenser, stirrer and thermometer. Heat was supplied by a heating mantle. The reaction mixture was refluxed at 83°C for 22 hours. After cooling the benzene was removed and the resultant product:

$$(HSCH_2CH_2\overset{\overset{O}{\|}}{C}OCH_2)_3CCH_2O\overset{\overset{O}{\|}}{C}CH_2CH_2SCH_2\overset{\overset{COOH}{|}}{C}HCH_2COOH$$

was confirmed by proton NMR.

Example 2

Using the same procedure as in Example 1, 65 g of itaconic acid and 262.8 g of tris(hydroxyethyl) isocyanurate tris(beta-mercaptopropionate) and 3.28 g of benzopinacol were charged to a flask containing 400 ml benzene. After refluxing for 24 hours followed by cooling, the benzene was separated from the product. The resultant product

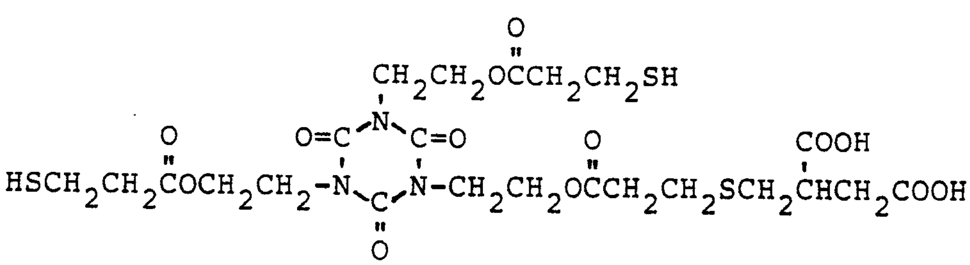

was confirmed by proton NMR.

Example 3

80 g of dipentaerythritol hexakis(beta-mercaptopropionate), 13 g itaconic acid and 0.09 g benzopinacol were charged to a 200 ml, 3-neck, round bottom flask equipped with glass stirrer and thermometer. The reaction mixture was heated at 125 - 130°C for 2 hours. After cooling, the viscous liquid reaction product, i., e.,

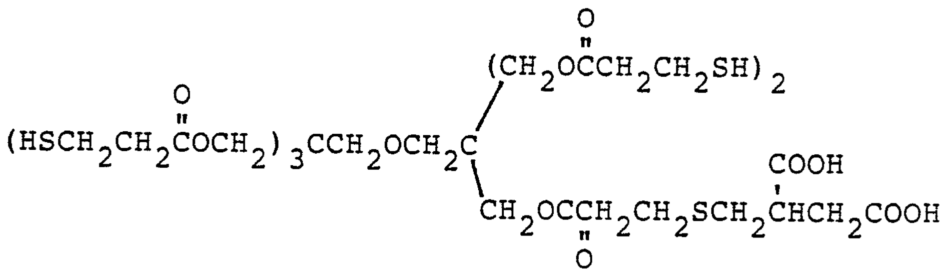

analyzed for 5.2 meq/g of SH and 2.1 meq/g of H+.

Example 4

80 g dipentaerythritol hexakis(beta-mercaptopropionate), 26 g itaconic acid and 0.11 g of benzopinacol were charged to a 200 ml, 3-neck, round bottom flask equipped with stirrer and thermometer. The reaction was heated with stirring to 113°C at which point it exothermed to 138°C where it was held for 1 hour and 10 minutes. After cooling, the viscous liquid product, was an adduct resulting from the addition of 2 moles of itaconic acid to 1 mole of the dipentaerythritol hexakis(beta-mercaptopropionate) as shown by the analysis for SH of 3.7 meq/g and H+ of 3.5 meq/g.

Example 5

The procedure of Example 4 was followed except that the reactants were 22.62 g of maleic acid, 100 g of pentaerythritol tetrakis(beta-mercaptopropionate) and 0.126 g of benzopinacol. After reacting the materials for 1 hour and 15 minutes at 140°C, the thick, grainy, white liquid product contained approximately 50% of:

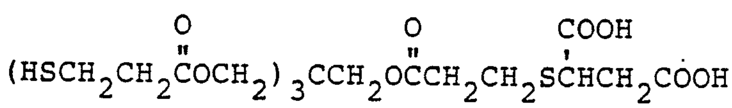

as shown by proton NMR.

Example 6

The procedure of Example 4 was followed except that the reactants were 50 g of pentaerythritol tetrakis(beta-mercaptopropionate), 12.66 g of itaconic acid and 0.13 g of 1,1-bis(t-butylperoxy)-3,3,5-trimethylcyclohexane. The reaction mixture was heated to 120°C and sustained at 120°C for 1 hour and 10 minutes with stirring. After cooling, the resultant liquid product was

$$(HSCH_2CH_2\overset{O}{\overset{\|}{C}}OCH_2)_3CCH_2O\overset{O}{\overset{\|}{C}}CH_2CH_2SCH_2\overset{COOH}{\overset{|}{C}}HCH_2COOH$$

as shown by proton and $^{13}$C NMR and analysis of 4.7 meq/g for SH and 2.8 meq/g for $H^+$.

Example 7

The procedure of Example 4 was followed except that the reactants were as follows:

60 g of pentaerythritol tetrakis(beta-mercaptopropionate), 8.42 g of glacial acrylic acid and 0.07 g of benzopinacol. The reaction was heated with stirring at 115°C to 120°C for 1½ hours. After cooling, the clear liquid product was

$$(HSCH_2CH_2\overset{O}{\overset{\|}{C}}OCH_2)_3CCH_2O\overset{O}{\overset{\|}{C}}CH_2CH_2SCH_2CH_2COOH$$

as shown by proton and $^{13}$C NMR and analysis of 5.5 meq/g for SH and 1.6 meq/g of $H^+$.

Example 8

50 g of pentaerythritol tetrakis(beta-mercaptopropionate), 12.66 g of itaconic acid and 0.13 g of alpha, alpha' azodiisobutyronitrile were charged to a 250 ml round bottom flask equipped with stirrer and thermometer. The reaction mixture was heated at 110°C -115°C for 1½ hours. After cooling, the viscous liquid product was

$$(HSCH_2CH_2\overset{O}{\overset{\|}{C}}OCH_2)_3CCH_2O\overset{O}{\overset{\|}{C}}CH_2CH_2SCH_2\overset{COOH}{\overset{|}{C}}HCH_2COOH$$

as shown by proton and $^{13}$C NMR and analysis of 4.7 meq/g for SH and 2.6 meq/g for $H^+$.

Example 9

80 g of trimethylolpropane tris(3-mercaptopropionate), 25.3 g of itaconic acid and 0.11 g of benzopinacol were charged to a 300 ml round bottom flask equipped with stirrer and thermometer. The flask was heated with stirring at 125°C - 130°C for 1½ hours. The resultant liquid product, i. e.,

$$(HSCH_2CH_2\overset{O}{\overset{\|}{C}}OCH_2)_2\overset{CH_2CH_3}{\overset{|}{C}}CH_2O\underset{O}{\underset{\|}{C}}CH_2CH_2SCH_2\overset{COOH}{\overset{|}{C}}CH_2COOH$$

analyzed for 3.8 meq/g of SH and 3.3 meq/g of $H^+$.

Example 10

2,514 g of pentaerythritol tetrakis(beta-mercaptopropionate) , 645.6 g itaconic acid and 3.16 g of benzopinacol were charged to a 3-liter resin kettle equipped with glass stirrer and thermometer. The reaction was heated to 124°C and exothermed to 146°C where it was maintained for 1 hour. After cooling, the resultant liquid product was

$$(HSCH_2CH_2\overset{O}{\overset{\|}{C}}OCH_2)_3CCH_2O\overset{O}{\overset{\|}{C}}CH_2CH_2SCH_2\overset{COOH}{\overset{|}{C}}HCH_2COOH$$

as shown by proton and $^{13}C$ NMR and analysis of 4.7 meq/g for SH and 2.7 meq/g for $H^+$.

Example 11

35 g of ethylene glycol bis(beta-mercaptopropionate), 18 g of itaconic acid and 0.05 g of benzopinacol were charged to a 250 ml round bottom flask equipped with stirrer and thermometer. The reaction mixture was heated at 120-130°C with stirring for 1½ hours. After cooling, the resultant product, i. e.,

$$HSCH_2CH_2\overset{O}{\overset{\|}{C}}OCH_2CH_2O\overset{O}{\overset{\|}{C}}CH_2CH_2SCH_2\overset{COOH}{\overset{|}{C}}HCH_2COOH$$

analyzed for 3.1 for SH and 4.9 for $H^+$.

Example 12

50 g of Capcure 3-800, commercially available from Diamond Shamrock, and which has the structure:

$$R[O(C_3H_6O)_nCH_2\overset{OH}{\overset{|}{C}}HCH_2SH]_3$$

where n is 1-2 and R is an aliphatic hydrocarbon was added along with 7.5 g itaconic acid and 0.06 g of benzopinacol to a 250 ml round bottom flask equipped with stirrer and thermometer. Capcure 3-800 was heated to 50°C before being charged to the flask. The reaction mixture was heated at 120°C -130°C with stirring for 1½ hours. After cooling, the resultant product, i. e.,

$$[HSCH_2\overset{OH}{\overset{|}{C}}HCH_2(OC_3H_6)_nO]_2RO(C_3H_6O)_nCH_2\overset{OH}{\overset{|}{C}}HCH_2SCH_2\overset{COOH}{\overset{|}{C}}HCH_2COOH$$

analyzed for 2.9 meq/g of SH and 1.8 meq/g of $H^+$.

cooling, the resultant product, i. e.,

$$[HSCH_2\overset{OH}{C}HCH_2(OC_3H_6)_nO]_2RO(C_3H_6O)_nCH_2\overset{OH}{C}HCH_2SCH_2\overset{COOH}{C}HCH_2COOH$$

analyzed for 2.9 meq/g of SH and 1.8 meq/g of $H^+$.

The following examples show the improved aqueous development and adhesion obtained by adding the thioether containing mercaptocarboxylic acids of the instant invention to a photocurable composition.

Example 13

A resin kettle was fitted with a stirrer, thermometer, drying tube and an addition funnel. 64.5 g of hydroxyethyl methacrylate, 69.5 g of hexamethylene diisocyanate, 0.2 g of hydroquinone monomethyl ether; 1.6 g of triphenylphosphite and 0.04 g of pyrogallol were added to this kettle. After heating the mixture to 35° -40°C, there was added 0.02 g of stannous octoate, and the mixture exothermed to 65°C. The temperature was maintained between 58 -63°C for 2½ hours at which time substantially all of the hydroxy groups were consumed and the NCO content reached 2.28 meg/g as determined by titration with dibutylamine. Thereafter, 37.2 g of polyethylene glycol with a molecular weight of 1,500 followed by 229 g of poly(ethylene glycol adipate) with a molecular weight of 1,000 were added along with 0.045 g of stannous octoate. After the temperature reached 70°C, there was added 0.06 g of stannous octoate and the reaction mixture was held at 65° -70°c for approximately 6 hours until all the isocyanate groups were consumed. To this reaction mixture was added 11.37 g of maleic anhydride and 0.4 g of hydroquinone and 2 g of dibutyl tin dilaurate. The reaction mixture was slowly heated up to 55°C to dissolve the maleic anhydride. The heating was continued and held at 70° -75°C for approximately 5 hours until the maleic anhydride was completely reacted, as indicated by the absence of 1,845 and 1,975 $cm^{-1}$ peaks in an IR spectrograph. The final product thus obtained was a solid at 25°C and had an acid content of 0.27 meg/g. This material will hereinafter be referred to as Prepolymer A.

Example 14

5 g of Prepolymer A from Example 13 were melted at 65°C and then mixed with 1.0g trimethylolpropane triacrylate, 0.12 g of benzophenone and 0.2 g of 2,2-dimethoxy-2-phenylacetophenone, commercially available from Ciba-Geigy under the tradename "Irgacure-651". The above formulation will hereinafter be referred to as "Photocurable Formulation A". 2.2 g of Photocurable Formulation A were admixed with 0.22 g of the reaction product from Example 10, which will be referred to as "Photocurable Formulation B". Using a drawbar, the formulations were drawn down on a clean epoxy fiberglass substrate laminated on both sides with copper. The coating thickness of the formulations was 1.0 to 1.5 mils. Each drawn down coating was imaged through a Stouffer wedge phototool for 10 seconds using a medium pressure mercury lamp (12 milliwatts/$cm^2$). The imaged boards were developed in a Circuit Systems Processor (conveyor speed 20"/min.) in a 0.7 to 0.8% aqueous sodium carbonate solution at 30°C. The developed boards were dried for 2 minutes in a 65°C forced air oven, then cooled at room temperature. The resultant Photocurable Formulation A had a residual scum on the copper surface which was not present on Photocurable Formulation B. In the Gardner cross hatch adhesion test carried out in accord with ASTM D 3359-74 using 3M 610 tape, 1" wide, the coating was completely removed from Photocurable Formulation A including areas around the cross hatch whereas in Photocurable Formulations B no coating was removed by the tape.

## Claims

1. Thioether containing mercaptocarboxylic acids which comprise the reaction product of a polythiol of the formula $(HS)_xR$ wherein R is an organic moiety with the valence of x and x is two or more and an unsaturated carboxylic acid selected from the group consisting of acrylic, methacrylic, itaconic and maleic, said acid being present in an

amount ranging from that sufficient to react with 1 up to (x minus 1) of the SH groups present in the polythiol.

2. A thioether containing mercaptocarboxylic acid according to claim 1 wherein the polythiol is pentaerythritol tetrakis(beta-mercaptopropionate), tris(hydroxyethyl)isocyanurate tris(beta-mercaptopropionate), dipentaerythritol hexakis (beta-mercaptopropionate), trimethylolpropane tris(3-mercaptopropionate), or ethylene glycol bis(beta-mercaptopropionate).

3. A thioether according to claim 1 or 2, wherein the amount of the said acid is such as to react with 1 SH group of the polythiol.

4. A process of forming a thioether containing mercaptocarboxylic acid which comprises reacting a polythiol of the formula $(HS)_xR$ wherein R is an organic moiety with the valence of x and x is two or more with an unsaturated carboxylic acid selected from the group consisting of acrylic, methacrylic, itaconic and maleic at a temperature in the range of about 40 to 200°C in the presence of a free radical initiator, said acid being present in an amount ranging from that sufficient to react with 1 up to(x minus 1) of the SH groups in the polythiol.

5. A process according to claim 4 wherein the reaction is carried out in a solvent.

6. A process according to claim 4 or 5 wherein the polythiol used is pentaerythritol tetrakis(beta-mercaptopropionate), tris(hydroxyethyl)isocyanurate tris(beta-mercapto propionate), dipentaerythritol hexakis (beta-mercaptopropionate), trimethylolpropane tris(3-mercaptopropionate), or ethylene glycol bis(beta-mercaptopropionate).

7. A process according to claim 4, 5 or 6 wherein the amount of the said acid is such as to react with 1 SH group of the polythiol.

8. A photocurable composition comprising a thioether containing mercaptocarboxylic acid as claimed in any of claims 1 to 3.

European Patent Office

# EUROPEAN SEARCH REPORT

Application number

EP 86 30 5240

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A- 663 201 (CARBIDE AND CARBON CHEMICALS CORP.)<br>--- | | C 07 C 149/20<br>C 07 D 251/34<br>G 03 F 7/10 |
| A | GB-A-2 036 000 (ARGUS CHEMICAL)<br>--- | | |
| A | DE-A-2 629 599 (KAO SOAP CO.)<br>--- | | |
| A | DE-A-1 918 342 (ICI)<br>----- | | |
| | | | TECHNICAL FIELDS SEARCHED (Int. Cl.4) |
| | | | C 07 C 149/00<br>C 07 D 251/00 |

The present search report has been drawn up for all claims

| Place of search | Date of completion of the search | Examiner |
|---|---|---|
| THE HAGUE | 27-10-1986 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82